# EUROPÄISCHE PATENTSCHRIFT

(11) **EP 1 799 183 B1**
(45) Veröffentlichungstag und Bekanntmachung des Hinweises auf die Patenterteilung: **24.04.2013**
(21) Anmeldenummer: 05794373.0
(22) Anmeldetag: 06.10.2005
(51) Int. Cl.: A61K 9/00, A61K 8/02, A61Q 11/00

(54) **SELBSTKLEBENDE ZAHNFOLIE**
SELF-ADHESIVE DENTAL FLOSS
FILM DENTAIRE AUTO-ADHESIF

(30) Priorität: 13.10.2004 DE 102004049740
(43) Veröffentlichungstag der Anmeldung: 27.06.2007
(73) Patentinhaber: LTS LOHMANN Therapie-Systeme AG, 56626 Andernach (DE)
(72) Erfinder: BOTZEM, Petra, 56626 Andernach (DE); LAUX, Wolfgang, 65582 Diez (DE)
(74) Vertreter: Schweitzer, Klaus
(86) Internationale Anmeldenummer: PCT/EP2005/010749
(87) Internationale Veröffentlichungsnummer: WO 2006/040059

(56) Entgegenhaltungen:
- WO-A-03/000216
- WO-A-2005/110344
- US-A- 5 747 017
- DATABASE WPI Section Ch, Week 200414 Derwent Publications Ltd., London, GB; Class A18, AN 2004-140515 XP002307581 & KR 2003 059 552 A (SK CHEM CO LTD) 10. Juli 2003 (2003-07-10)
- PATENT ABSTRACTS OF JAPAN Bd. 1999, Nr. 14, 22. Dezember 1999 (1999-12-22) & JP 11 240816 A (OKA HIRONORI), 7. September 1999 (1999-09-07)

## Beschreibung

Die vorliegende Erfindung betrifft folienförmige, an den Zähnen, dem Zahnfleisch oder der Mundschleimhaut haftklebende Zubereitungen, die für eine kosmetische oder medizinische Behandlung der Zähne, insbesondere der Zahnhälse, und des Zahnfleisches geeignet sind. Ferner betrifft die vorliegende Erfindung ein Verfahren zur Herstellung derartiger folienförmiger Zubereitungen.

Produkte zur Mundreinigung und Wirkstoffzufuhr sind in Form von Pasten, Cremes oder Mundspüllösungen seit langem bekannt und auf dem Markt. Diese Produkte schließen reinigende, vor Karies schützende, desensibilisierende sowie bleichende Zahnpasten und Cremes ein. Des weiteren sind Cremes, Gele und Salben bekannt, die im Mundbereich zur Behandlung örtlicher Erkrankungen und Störungen verwendet werden, wie zum Beispiel entzündungshemmende, schmerzstillende und/oder kräftigende Mittel.

Seit einigen Jahren sind auch folien-, flächen- oder streifenförmige orale Anwendungsformen im Handel erhältlich, mit denen kosmetische oder therapeutische Ziele verfolgt werden. Zu diesen Anwendungsformen zählen beispielsweise die Listerine Pocket-Paks^{®} der Firma Pfizer oder die "Zahn-Weiß-Streifen" der Firma Procter & Gamble. Derartige Anwendungsformen basieren in der Regel auf aus wasserlöslichen Polymeren zusammengesetzten Folien, die bei Anwendung relativ schnell zerfallen und insofern für eine länger andauernde Behandlung ungeeignet sind.

Darüber hinaus sind aus mehreren Schichten bestehende Produkte bekannt, wie beispielsweise die in US 6,582,708 offenbarten, von denen jedoch nur eine Schicht flexibel ist und die mit einer Gesamtdicke von bis zu 3 mm im Mund als lästig und unangenehm empfunden werden.

KR2003059552 offenbart eine haftklebenden Zahnbleichungsfolie, bei der das Bleichmittel in einem Polymer dispergiert ist. Das Polymer kann unter underem ein copolymer aus Methylvinylether und Maleinsänreanhydrid sein.

Bei den bei der Herstellung der bekannten folienförmigen, Anwendungsformen verwendeten Rezepturen und Verfahren wird überwiegend von Wasser oder wasserhaltigen alkoholischen Polymerlösungen ausgegangen. Nachteilig dabei ist, dass eine vollständig gelöste Formulierung von lipophilen, schwer wasserlöslichen oder wasserempfindlichen Wirkstoffen nur sehr eingeschränkt möglich ist.

Aufgabe der vorliegenden Erfindung war es daher, ein ein- oder mehrschichtiges, wasserlösliches oder zumindest teilweise in Wasser quellfähiges, folienförmiges Material bereitzustellen, das gut an Zähnen, dem, Zahnfleisch oder der Mundschleimhaut haftet, sich als Wirkstoffträger eignet und nicht bereits innerhalb weniger Sekunden zerfällt. Insbesondere soll ein derartiges folienförmiges Material bereit gestellt werden, mit dem wasserunlösliche oder wasserempfindliche Wirkstoffe verabreicht werden können.

Die Aufgabe wird durch das Bereitstellen einer ein- oder mehrschichtigen, haftklebenden, folienförmige Zubereitung gelöst, die zumindest ein Polymethylvinylether-Maleinsäureanhydrid-Copolymer und zumindest ein Copolymer des Polyvinylacetats mit Vinylalkoholestern von Fettsäuren, sowie zumindest einen kosmetischen und/oder pharmazeutischen Wirkstoff enthält.

Vorzugsweise beträgt der Gehalt an Polymethylvinylether-Maleinsäureanhydrid-Copolymer bei den folienförmigen, haftklebenden zubereitungen 5 bis 35 Gew.-%.

Der Gehalt an dem oder den Copolymer (en) des Polyvinylacetats mit Vinylalkoholestern von Fettsäuren beträgt bei den folienförmigen, haftklebenden Zubereitungen vorzugsweise 4 bis 35 Gew.-%.

Die folienförmigen, haftklebenden Zubereitungen weisen zudem einen Gehalt an zumindest einem kosmetischen und/oder pharmazeutischen Wirkstoff auf, der sich nach der Natur der Erkrankung oder den kosmetischen Bedürfnissen richtet. Vorzugsweise handelt es sich bei dem Wirkstoff um einen schwer wasserlöslichen Wirkstoff oder um einen wasserempfindlichen Wirkstoff.

Unter schwer wasserlöslichen Wirkstoffen werden Wirkstoffe mit einer Löslichkeit in Wasser von weniger als 1 g/l verstanden. Dazu zählen beispielsweise Griseofulvin, Estradiol und Vitamin D3.

Zu den wasserempfindlichen Wirkstoffen werden die Wirkstoffe gezählt, bei denen eine wässrige Lösung dieses Wirkstoffs innerhalb von 24 Stunden bei Raumtemperatur mehr als 0,1 % ihres Wirkstoffgehalts durch Abbau des Wirkstoffs verliert, Zu den wasserempfindlichen Wirkstoffen gehören beispielsweise Acetylsalicylsäure und Jod.

In einer besonders bevorzugten Ausführungsform weist die folienförmige, haftklebende Zubereitung zusätzlich einen Gehalt an Carboxymethylcellulose und/oder eines ihrer Salze auf. Der Gehalt an Carboxymethylcellulose oder ihres Salzes beträgt in der Zubereitung bevorzugt 5 bis 35 Gew.-%.

Für die Herstellung der an Zähnen, dem Zahnfleisch oder der Mundschleimhaut haftklebenden, folienförmigen Zubereitung werden die verschiedenen, wasserlöslichen und/oder in wasser quellfähigen Polymere mit zumindest einem Wirkstoff in Lösungen, die organische Lösungsmittel als Hauptkomponente enthalten, gemischt. Mit diesem Gemisch wird eine Unterlage beschichtet und gegebenenfalls unter Wärmeeinwirkung getrocknet.

Die Vorteile der Erfindung stellen sich insbesondere bei der Verwendung von Gemischen aus Polymethylvinylether-Maleinsäureanhydrid-Copolymeren mit Copolymeren des Polyvinylacetats mit Vinylalkoholestern von Fettsäuren ein.

Vorzugsweise beträgt der Gehhalt an Polymethylvinylether-Maleinsäureanhydrid-Copolymer 5 bis 35 Gew.-% und der Gehalt an dem oder den Copolymer(en) des Polyvinylacetats mit Vinylalkoholestern von Fettsäuren 4 bis 35 Gew.-%, jeweils bezogen auf den festen Anteil.

Als erfindungsgemäß besonders geeignet haben sich die unter dem Namen "Gantrez®" von der Firma ISP vertriebenen Polymethylvinylether-Maleinsäureanhydrid-Copolymere und die als "VINNAPAS®" bezeichneten Polyvinylacetate bzw. deren Copolymere mit Vinylalkoholestern von Fettsäuren der Firma Wacker Polymer Systems, Burghausen, herausgestellt.

Als organische Lösungsmittel, die bei dem erfindungsgemäßen Verfahren als Hauptkomponente in der Lösung enthalten sind, können die Lösungsmittel verwendet werden, in denen sich die Polymethylvinylether-Maleinsäureanhydrid-Copolymere und Copolymere des polyvinylacetats mit Vinylalkoholestern von Fettsäuren lösen. Bevorzugt werden von diesen organischen Lösungsmitteln diejenigen verwendet, die sich auch mit Wasser mischen, da durch die Zugabe kleiner Mengen Wasser, d. h. von 1 bis 5 Gew.-%. die Lösungsfähigkeit für die Polymere sowie den Wirkstoff/die Wirkstoffe den jeweiligen Erfordernissen anpassbar ist. Ganz besonders bevorzugt werden die organischen Lösungsmittel, die eine Zugabe des Wirkstoffs in Ethanol oder einem Ethanol-Wasser-Gemisch gelöst erlauben. Das besonders bevorzugte organische Lösungsmittel wird aus der Gruppe ausgewählt, die Methylethylketon, Ethylacetat, Ethanol, Aceton und deren Mischungen umfasst.

Die Konsistenz und/oder Klebrigkeit der resultierenden filmförmigen Zubereitung lässt sich durch eine Veränderung des Mischungsverhältnisses von Ethanol zu Methylethylketon den an das Produkt gestellten Erfordernissen anpassen. Als vorteilhaft haben sich Lösungsmittelgemische mit einem Anteil an Ethanol von 5 bis 20 Ges,-%, bezogen auf das gesamte Lösungsmittel, erwiesen.

Zur Herstellung einer bevorzugten Ausführungsform, die eine besonders erfolgreiche Verklebung der folienförmigen, Zubereitung mit den Zähnen, dem Zahnfleisch und/oder der Mundschleimhaut ermöglicht, wird der Polymermischung Carboxymethylcellulose und/oder zumindest eines ihrer Salze zugesetzt.

Vorzugsweise wird der polymermischung 5 bis 35 Gew.-% Carboxymethylcellulose oder zumindest eines ihrer Salze, bezogen auf den festen Anteil, zugesetzt.

Zur Herstellung der erfindungsgemäßen. Zubereitungen werden zumindest ein Polymethylvinylether-Maleisäureanhydrid-Copolymer und zumindest ein Copolymer des Vinylacetats mit Vinylalkoholestern von Fettsäuren zusammen mit zumindest einem Wirkstoff und gegebenenfalls Carboxymethylcellulose und/oder zumindest eines ihrer Salze, in einer Lösung gemischt, welche ein oder mehrere organische Lösungsmittel als Hauptkomponente enthält. Die so erhaltene Masse wird flüssig auf eine Unterlage ausgetragen und durch Trocknen vom Lösungsmitteigemisch befreit, so dass eine Folie mit einer Schichteicke von 4 bis 2000 µm, vorzugsweise von 40 bis 500 µm und besonders bevorzugt von 60 bis 250 µm, erhalten wird.

Mehrschichtige, haftklebende folienförmige Zubereitungen, mit denen zum Beispiel eine verbesserte Adhäsion der Zubereitung auf Zähnen, errecht werden kann, können beispielsweise durch Hitzielaminierung, durch Beschichten bereits vorhandener Schichten mit weiteren läsungsmittelhaltigen Massen, wie zuvor für die Herstellung einer einschichtigen Folie beschrieben, oder anderen, dem Fachmann bekannten verfahren hergestellt werden.

Aus der Folie bzw. dem Laminat werden Stücke in dem jeweiligen Zweck angemessener Größe ausgestanzt.

Aus dem erfindungsgemäßen Verfahren resultieren flexible, folienförmige Zubereitungen, die auf Zähnen, dem Zahnfleisch oder der Mundschleimhaut haften, nicht bereits innerhalb weniger Sekunden zerfallen und mit denen wasserunlösliche und sogar wasserempfindliche Wirkstoffe verabreicht werden können. Diese Zubereitungen können vorteilhafterweise zur medizinischen oder kosmetischen Behandlung verwendet werden.

### Beispiel:

5 g einer 33,3 %-igen Lösung (Gew.-%) eines Polymers auf Basis von Vinylacetat in Methylethylketon und 3 g einer 15 %-igen Lösung (Gew.-%) von Polymethylvinylether-Maleinsäureanhydrid (= Copolymer aus Methylvinylether und Maleinsäureanhydrid)in Ethylacetat wurden zusammengegeben und homogenisiert. Es entstand eine Lösung, die mit unterschiedlicher Strichstärke ausgestrichen und anschließend getrocknet wurde. Es resultierten flexible Folien, die sowohl an der Mundschleimhaut als auch an den Zähnen hafteten.

## Patentansprüche

1. Ein- oder mehrschichtige, folienförmige, haftklebende Zubereitung zur Anwendung im Mundraum, insbesondere auf den Zähnen, enthaltend zumindest ein Polymethylvinylether-Maleinsäureanhydrid-Copolymer, und zumindest ein Copolymer des Polyvinylacetats mit Vinylalkoholestern von Fettsäuren, sowie zumindest einen kosmetischen und/oder pharmazeutischen Wirkstoff.

2. Zubereitung nach Anspruch 1, **dadurch gekennzeichnet, dass** der Gehalt an Polymethylvinylether-Maleinsäureanhydrid-Copolymer 5 bis 35 Gew.-% beträgt.

3. Zubereitung nach Anspruch 1 oder 2, **dadurch gekennzeichnet, dass** der Gehalt an dem oder den Copolymer(en) des Polyvinylacetats mit Vinylalkoholestern von Fettsäuren 4 bis 35 Gew. -% beträgt.

4. Zubereitung nach einem der vorangehenden Ansprüche, **dadurch gekennzeichnet, dass** der Wirkstoff schwer wasserlöslich oder wasserempfindlich ist.

5. Zubereitung nach einem der vorangehenden Ansprüche, **dadurch gekennzeichnet, dass** sie einen zusätzlichen Gehalt an Carboxymethylcellulose und/oder zumindest einem ihrer Salze aufweist.

6. Zubereitung nach Anspruch 5, **dadurch gekennzeichnet, dass** der Gehalt an Carboxymethylcellulose und/oder ihres Salzes 5 bis 35 Gew.-% beträgt.

7. Zubereitung nach einem der vorangehenden Ansprüche, **dadurch gekennzeichnet, dass** die Zubereitung eine Schichtdicke von 4 bis 2000 µm, vorzugsweise von 40 bis 500 µm und besonders bevorzugt von 60 bis 250 µm, aufweist.

8. Verfahren zur Herstellung einer Zubereitung nach Anspruch 1, **gekennzeichnet durch**
- Mischen von zumindest einem Polymethylvinylether-Maleinsäureanhydrid-Copolymer, und zumindest einem Copolymer des Polyvinylacetats mit Vinylalkoholestern von Fettsäuren sowie zumindest einem kosmetischen und/oder pharmazeutischen Wirkstoff in einem Lösungsmittel, welches ein oder mehrere organische Lösungsmittel als Hauptbestandteil enthält;
- Beschichten einer Unterlage mit dem so erhaltenen Gemisch, und
- Entfernen des Lösungsmittels **durch** Trocknen.

9. Verfahren nach Anspruch 8, **dadurch gekennzeichnet, dass** der Gehalt an Polymethylvinylether-Maleinsäureanhydrid-Copolymer 5 bis 35 Gew.-%, bezogen auf den festen Anteil, beträgt.

10. Verfahren nach Anspruch 8 oder 9, **dadurch gekennzeichnet, dass** der Gehalt an dem oder den Copolymer(en) des Polyvinylacetats mit Vinylalkoholestern von Fettsäuren 4 bis 35 Gew.-%, bezogen auf den festen Anteil, beträgt.

11. Verfahren nach Anspruch 8, **dadurch gekennzeichnet, dass** das Lösungsmittel 1 bis 5 Gew.-% Wasser enthält.

12. Verfahren nach einem der Ansprüche 8 bis 11, **dadurch gekennzeichnet, dass** der Wirkstoff schwer wasserlöslich oder wasserempfindlich ist.

13. Verfahren nach einem der Ansprüche 8 bis 12, **dadurch gekennzeichnet, dass** das organische Lösungsmittel aus der Gruppe ausgewählt wird, die Methylethylketon, Ethylacetat, Ethanol, Aceton und deren Mischungen umfasst.

14. Verfahren nach Anspruch 13, **dadurch gekennzeichnet, dass** das organische Lösungsmittel eine Mischung von Ethanol und Methylethylketon ist.

15. Verfahren nach Anspruch 14, **dadurch gekennzeichnet, dass** die Mischung einen Anteil an Ethanol von 5 bis 20 Gew.-% aufweist.

16. Verfahren nach einem der Ansprüche 8 bis 15, **dadurch gekennzeichnet, dass** der Masse vor Auftragen auf die Unterlage Carboxymethylcellulose und/oder zumindest eines ihrer Salze zugesetzt wird.

17. Verfahren nach Anspruch 16, **dadurch gekennzeichnet, dass** der Gehalt an Carboxymethylcellulose und/oder ihrer Salze 5 bis 35 Gew.-%, bezogen auf den festen Anteil, beträgt.

18. Verfahren nach einem der Ansprüche 8 bis 17, **dadurch gekennzeichnet, dass** nach dem Trocknen der Beschichtung eine oder mehrere weitere Schichten durch Hitzelaminierung oder Beschichten der vorhandenen Schicht(en) mit einer oder mehreren lösungsmittelhaltigen Massen auf die bereits vorhandene(n) Schicht(en) aufgebracht werden.

19. Verfahren nach einem der Ansprüche 8 bis 18, **gekennzeichnet durch** das Auftragen der Masse auf die Unterlage, so dass nach Trocknen eine Schichtdicke von 4 bis 2000 µm, vorzugsweise von 40 bis 500 µm und besonders bevorzugt von 60 bis 250 µm, erhalten wird.

20. Verwendung einer Zubereitung nach einem der Ansprüche 1 bis 7 zur kosmetischen Behandlung im Mundraum, insbesondere der Zähne oder des Zahnfleisches.

21. Zubereitung nach einem der Ansprüche 1 bis 7 zur Verwendung als Arzneimittel.

## Claims

1. Single-layer or multi-layer, film-like, pressure-sensitive adhesive preparation for use in the oral cavity, in particular on the teeth, containing at least one poly(methyl vinyl ether-maleic anhydride)copolymer and at least one copolymer of poly(vinyl acetate) with vinyl alcohol esters of fatty acids, and at least one cosmetic and/or pharmaceutical active substance.

2. Preparation according to Claim 1, **characterized in that** the poly(methyl vinyl ether-maleic anhydride)copolymer content is 5 to 35 % by weight.

3. Preparation according to Claim 1 or 2, **characterized in that** the content of the copolymer(s) of poly(vinyl acetate) with vinyl alcohol esters of fatty acids is 4 to 35 % by weight.

4. Preparation according to one of the preceding claims, **characterized in that** the active substance is sparingly soluble in water or water-sensitive.

5. Preparation according to one of the preceding claims, **characterized in that** it has an additional content of carboxymethylcellulose and/or at least one of its salts.

6. Preparation according to Claim 5, **characterized in that** the content of carboxymethylcellulose and/or its salts is 5 to 35 % by weight.

7. Preparation according to one of the preceding claims, **characterized in that** the preparation has a layer thickness of 4 to 2000 µm, preferably 40 to 500 µm and especially preferably 60 to 250 µm.

8. Process for preparing a preparation according to Claim 1, **characterized in that**
- at least one poly(methyl vinyl ether-maleic anhydride copolymer and at least one copolymer of poly(vinyl acetate) with vinyl alcohol esters of fatty acids and at least one cosmetic and/or pharmaceutical active substance are mixed in a solvent which contains one or more organic solvents as the main constituent;
- a substrate is coated with the resulting mixture and
- the solvent is removed by drying.

9. Process according to Claim 8, **characterized in that** the poly(methyl vinyl ether-maleic anhydride)copolymer content is 5 to 35 % by weight, based on the solid fraction.

10. Process according to Claim 8 or 9, **characterized in that** the content of the copolymer (s) of poly(vinyl acetate) with vinyl alcohol esters of fatty acids is 4 to 35 % by weight, based on the solid fraction.

11. Process according to Claim 8, **characterized in that** the solvent contains 1 to 5 % by weight of water.

12. Process according to one of Claims 8 to 11, **characterized in that** the active substance is sparingly soluble in water or water-sensitive.

13. Process according to one of Claims 8 to 12, **characterized in that** the organic solvent is selected from the group which comprises methyl ethyl ketone, ethyl acetate, ethanol, acetone and their mixtures.

14. Process according to Claim 13, **characterized in that** the organic solvent is a mixture of ethanol and methyl ethyl ketone.

15. Process according to Claim 14, **characterized in that** the mixture contains an ethanol fraction of 5 to 20 % by weight.

16. Process according to one of Claims 8 to 15, **characterized in that** carboxymethylcellulose and/or at least one of its salts is added to the composition before it is applied to the substrate.

17. Process according to Claim 16, **characterized in that** the content of carboxymethylcellulose and/or its salts is 5 to 35 % by weight, based on the solid fraction.

18. Process according to one of Claims 8 to 17, **characterized in that**, after the coating has dried, one or more further layers are applied to the already existing layer(s) by heat lamination or by coating the existing layer(s) with one or more solvent-containing compositions.

19. Process according to one of Claims 8 to 18, **characterized in that** the composition is applied to the substrate such that, after drying, a layer thickness of 4 to 2000 µm, preferably 40 to 500 µm and especially preferably 60 to 250 µm is obtained.

20. Use of a preparation according to one of Claims 1 to 7 for cosmetic treatment in the oral cavity, in particular of the teeth or the gums.

21. Preparation according to one of Claims 1 to 7 for use as a medicament.

## Revendications

1. L'invention concerne une préparation adhésive de type film, à une ou plusieurs couches, destinée à être utilisée dans la cavité buccale, en particulier sur les dents. Cette préparation contient au moins un copolymère polyéther méthylvinylique-anhydride maléique et au moins un copolymère du polyacétate de vinyle avec des esters d'alcool vinylique d'acides gras, ainsi qu'au moins un principe actif cosmétique et/ou pharmaceutique.

2. Préparation selon la revendication 1, **caractérisé en ce que** le contenu de copolymère polyéther méthylvinylique-anhydride maléique est de 5 à 35% en poids.

3. Préparation selon les revendications 1 ou 2, **caractérisée en ce que** le contenu en copolymère ou copolymères de polyacétate de vinyle avec des esters d'alcool vinylique d'acides gras est de 4 à 35% en poids.

4. Préparation selon une des revendications précédentes, **caractérisée en ce que** le principe actif est difficilement soluble dans l'eau ou est sensible à l'eau.

5. Préparation selon une des revendications précédentes, **caractérisée en ce qu'**elle présente un contenu supplémentaire en carboxyméthylcellulose et/ou au moins en un de ses sels.

6. Préparation selon la revendication 5, **caractérisée en ce que** le contenu en carboxyméthylcellulose et/ou de ses sels est de 5 à 35% en poids.

7. Préparation selon une des revendications précédentes, **caractérisée en ce que** la préparation présente une épaisseur de couche allant de 4 à 2000 µm, de préférence de 40 à 500 µm et idéalement de 60 à 250 µm.

8. Procédé servant à produire une préparation selon la revendication 1, caractérisé per
- le mélange d'un copolymère polyéther méthylvinylique-anhydride maléique et au moins d'un copolymère de polyacétate de vinyle avec des esters d'alcool vinylique d'acides gras ainsi qu'au moins d'un principe actif cosmétique ou pharmaceutique dans un solvant, lequel contient un ou plusieurs solvants organiques en tant que constituant principal;
- revêtement d'un support avec le mélange ainsi obtenu et
- retrait du solvant par séchage.

9. Procédé selon la revendication 8, **caractérisé en ce que** le contenu en copolymère polyéther méthylvinylique-anhydride maléique est de 5 à 35 % en poids par rapport à la partie solide.

10. Procédé selon les revendications 8 ou 9, **caractérisé en ce que** le contenu en copolymère ou copolymères de polyacétate de vinyle avec des esters d'alcool vinylique d'acides gras est de 4 à 35% en poids, par rapport à la partie solide.

11. Procédé selon la revendication 8, **caractérisé en ce que** le solvant contient de 1 à 5% en poids d'eau.

12. Procédé selon une des revendications de 8 à 11, **caractérisé en ce que** le principe actif est difficilement soluble dans l'eau ou est sensible à l'eau.

13. Procédé selon une des revendications de 8 à 12, **caractérisé en ce que** le solvant organique est choisi à partir du groupe qui comprend du méthyléthylcétone, de l'acétate d'éthyle, de l'éthanol, de l'acétate et leurs mélanges.

14. Procédé selon la revendication 13, **caractérisé en ce que** le solvant organique est un mélange d'éthanol et de méthyléthylcétone.

15. Procédé selon la revendication 14, **caractérisé en ce que** le mélange présente une partie d'éthanol allant de 5 à 20% en poids.

16. Procédé selon une des revendications de 8 à 15, **caractérisé en ce que** du carboxyméthylcellulose et/ou au moins un de ses sels est ajouté à la masse avant l'application sur le support.

17. Procédé selon la revendication 16, **caractérisé en ce que** le contenu de carboxyméthylcellulose et/ou ses sels est de 5 à 35% en poids, par rapport à la partie solide.

18. Procédé selon une des revendications de 8 à 17, **caractérisé en ce que**, après le séchage du revêtement, une ou plusieurs autres couches sont appliquées sur les couches actuellement existantes par laminage à chaud ou par revêtement des couches existantes avec une ou plusieurs masses à base de solvant.

19. Procédé selon une des revendications de 8 à 18, **caractérisé en ce que** par l'application de la masse sur le support de sorte qu'après le séchage l'on obtient une épaisseur de couche allant de 4 à 2000 µm, de préférence de 40 à 500 µm et idéalement de 60 à 250 µm.

20. Utilisation d'une préparation selon une des revendications de 1 à 7 destinée au traitement cosmétique de la cavité buccale, en particulier des dents ou des gencives.

21. Préparation selon une des revendications de 1 à 7 destinée à être utilisée en tant que remède médicale.
